(19) **Europäisches Patentamt / European Patent Office / Office européen des brevets**

(11) **EP 3 558 125 B1**

(12) **FASCICULE DE BREVET EUROPEEN**

(45) Date de publication et mention
de la délivrance du brevet:
**23.12.2020 Bulletin 2020/52**

(21) Numéro de dépôt: **18708160.9**

(22) Date de dépôt: **24.01.2018**

(51) Int Cl.:
*A61B 6/00* *(2006.01)* *A61B 5/00* *(2006.01)*
*G06T 7/00* *(2017.01)*

(86) Numéro de dépôt international:
**PCT/FR2018/050164**

(87) Numéro de publication internationale:
**WO 2018/138435 (02.08.2018 Gazette 2018/31)**

(54) **PROCÉDÉ ET DISPOSITIF D'ESTIMATION DE GRAISSE VISCÉRALE D'UN CORPS VIVANT**

VERFAHREN UND VORRICHTUNG ZUR SCHÄTZUNG DES VISZERALEN FETTS EINES LEBENDEN KÖRPERS

METHOD AND DEVICE FOR ESTIMATING THE VISCERAL FAT OF A LIVING BODY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.01.2017 FR 1750575**

(43) Date de publication de la demande:
**30.10.2019 Bulletin 2019/44**

(73) Titulaire: **Diagnostic Medical Systems**
**34130 Mauguio (FR)**

(72) Inventeur: **GEITNER, Mickael**
**30035 Nîmes (FR)**

(74) Mandataire: **Cornuejols, Marine Sophie Cassiopi**
**230, avenue de l'Aube Rouge**
**34170 Castelnau-le-Lez (FR)**

(56) Documents cités:
**WO-A1-2016/138262 US-A1- 2011 311 122**
**US-A1- 2015 374 291 US-B1- 8 300 911**

**Description**

DOMAINE TECHNIQUE DE L'INVENTION

La présente invention vise un procédé et un dispositif d'estimation de graisse viscérale d'un corps vivant. Elle s'applique, notamment, à l'estimation de la quantité de graisse viscérale d'un corps vivant suite à une imagerie médicale pour laquelle la graisse viscérale est indifférenciable de la graisse sous-cutanée, telle une image par absorptiométrie bi-photonique.

ETAT DE LA TECHNIQUE

L'obésité est un excès de graisse dans un corps vivant qui atteint un grand nombre de personnes mondialement. Elle est l'une des causes d'invalidité, de mortalité et de coût sanitaire les plus élevées. L'obésité est définie comme un excès de tissus adipeux. La métabolisation de la graisse ainsi que sa localisation dans le corps humain sont complexes mais, celle-ci est généralement localisée autour des hanches, des cuisses, des fesses et dans la région androïde. Dans cette dernière, les tissus adipeux peuvent être dissociés en deux régions distinctes, une graisse dite sous-cutanée (en anglais, « subcutaneous adipose tissue » d'acronyme « SAT ») et une graisse dite viscérale (en anglais, « visceral adipose tissue » d'acronyme « VAT »).

La graisse sous-cutanée est un feuillet d'adipocytes directement localisé sous la peau. Malgré son aspect non esthétique, la graisse sous-cutanée n'est pas dangereuse pour la santé, à l'inverse de la graisse viscérale. En effet, des études ont montré que la quantité de graisse viscérale était fortement corrélée aux risques de maladie cardiovasculaire, c'est-à-dire avec l'évolution des facteurs de risque tels que les lipoprotéines de haute densité et de basse densité, de triglycérides, d'hypertension ou de quantité de glucose dans le sang. Ainsi, l'estimation de la graisse viscérale est un point clé dans la prévention de maladies cardiovasculaires.

Actuellement, différentes méthodes sont utilisées afin d'estimer la quantité de graisse : les balances à impédance, la méthode des plis de graisse, les équations de Deurenberg, etc. Néanmoins, ces méthodes ne permettent pas d'obtenir des résultats reproductibles. En effet, pour la plupart, ceux-ci dépendent des conditions de mesure, voire de l'opérateur. Ainsi, de nos jours, seulement deux méthodes de mesure s'affichent comme références dans l'estimation des masses graisseuses sous-cutanée et viscérale : l'imagerie par résonance magnétique (d'acronyme « IRM ») et la tomodensitométrie (en anglais, « CT-scan » pour « computerized tomography »). Cependant, l'accès à la quantité de graisse viscérale sur ces examens n'est pas trivial et nécessite fréquemment une intervention humaine. De plus, la segmentation des composants du corps vivant nécessite l'utilisation de seuils sensibles, dont une petite variation peut induire de grandes perturbations dans les résultats, rendant ceux-ci fortement dépendants de l'opérateur. A cela s'ajoute le fait qu'une fois la graisse segmentée, il faut dissocier la graisse viscérale de la graisse sous-cutanée, ce qui reste manuel et très peu reproductible. Enfin, le coût élevé de l'imagerie par résonance magnétique ainsi que la forte exposition au rayonnement ionisant de la tomodensitométrie empêchent l'utilisation de ces techniques d'imagerie plusieurs fois par an et ainsi interdisent le suivi régulier d'un corps vivant, humain ou animal, par exemple.

Les méthodes conventionnelles permettant d'estimer la graisse viscérale reposent sur des modèles simples cherchant à approximer géométriquement la cavité abdominale, et, par extension, la distribution de graisse sous-cutanée. Néanmoins, ces méthodes ne sont pas suffisamment efficaces et ne peuvent être basées sur des images d'absorptiométrie, même bi-photoniques. En effet, les images d'absorptiométrie bi-photonique sont des projections bidimensionnelles de corps. Ainsi, les informations sur la répartition tridimensionnelle des tissus n'est pas accessible. Cela implique que sur les images d'absorptiométrie bi-photonique, la graisse viscérale et la graisse sous-cutanée ne sont pas séparément quantifiables et qu'il faut alors faire des hypothèses sur leur répartition impliquant une estimation systématiquement erronée.

Une première méthode, décrite dans le brevet américain US 9,179,873 consiste à approximer la coupe transverse de l'enveloppe externe de l'abdomen du corps humain par une ellipse, puis, d'approximer la forme de la cavité abdominale, renfermant les organes et la graisse viscérale, par une seconde ellipse. En faisant l'hypothèse que la répartition de graisse sous-cutanée est uniforme entre ces deux ellipses il est alors possible de soustraire la quantité de graisse sous-cutanée modélisée à la quantité de graisse totale et d'en déduire la quantité de graisse viscérale comprise dans une coupe. Le volume de graisse viscérale dans l'abdomen peut être obtenue en traitant ainsi plusieurs coupes dans le volume de l'abdomen.

Une seconde méthode, décrite dans le brevet américain US 8,483,458 consiste à utiliser différentes méthodes d'estimation de la graisse viscérale en séparant la région abdominale en une section supérieure et une section inférieure, et, pour chacune d'elles, dissocier une portion postérieure et une portion antérieure. Bien que cette méthode semble plus précise que la simple utilisation d'ellipses, elle reste néanmoins une approximation géométrique du corps humain. Il est alors compréhensible que des méthodes basées sur de telles approximations ne puissent être suffisamment précises car celles-ci ne prennent pas en compte des paramètres physiologiques tels que l'âge ou le sexe du corps vivant, alors qu'il est reconnu que la métabolisation de la graisse dépend de ces deux paramètres.

Une autre méthode décrite dans le brevet américain US 8,300,911, consiste à acquérir une image planaire et déterminer d'au moins une courbe représentative de la projection de la graisse totale dans l'image.

OBJET DE L'INVENTION

La présente invention vise à remédier à tout ou partie de ces inconvénients. Notamment, la présente invention vise à effectuer une approximation, par parties, de la courbe représentative de la projection de graisse sous-cutanée d'un corps vivant par une fonction afin d'estimer la graisse viscérale d'un corps vivant.

A cet effet, selon un premier aspect, la présente invention vise un procédé d'estimation de graisse viscérale d'un corps vivant, qui comporte les étapes suivantes :

- acquisition d'au moins une image planaire représentative d'une composition corporelle comportant l'abdomen du corps vivant,
- détermination d'au moins une courbe représentative de la projection de la graisse totale dans l'image acquise suivant une coupe de l'image acquise,

pour chaque coupe :

- identification d'au moins quatre points d'intérêts définis tels que :

  - le premier point d'intérêt et le quatrième point d'intérêt correspondent au passage d'une valeur de graisse totale nulle à une valeur non nulle,
  - le deuxième point d'intérêt et le troisième point d'intérêt représentent un maximum local et
  - les courbes entre le premier point d'intérêt et le deuxième, et entre le troisième point d'intérêt et le quatrième, sont sensiblement des parties de projections d'une ellipse,

- définition d'au moins deux intervalles entre le deuxième et le troisième point d'intérêt de la courbe,
- pour chaque intervalle, estimation d'une fonction d'approximation de la graisse sous cutanée en fonction des variations attendues de la courbe représentative de la quantité de graisse totale et
- calcul de la quantité de graisse viscérale comportant la soustraction de la fonction d'approximation de la graisse sous-cutanée à la courbe représentative de la projection de la graisse totale.

Grâce à ces dispositions, les résultats concernant la quantité de graisse viscérale peuvent être utilisés dans le suivi régulier d'un corps vivant afin d'évaluer la progression de la proportion de graisse viscérale et de graisse sous-cutanée lors d'une période de régime, de traitement ou d'activité physique. Un suivi permet l'adaptation du régime, d'un traitement ou de l'activité physique requise.

Le procédé objet de l'invention s'affranchit notamment d'aspects géométriques de modélisation interne du corps d'un corps vivant en utilisant des répartitions statistiques de projections de graisse et cela, en fonction de paramètres physiologiques. Ainsi, le procédé est dissocié d'aspects géométriques.

Dans des modes de réalisation, sur chaque intervalle, la fonction d'approximation est une composition d'au moins une fonction polynomiale.

Ces modes de réalisation permettent d'obtenir une fonction d'approximation la plus proche possible de la courbe représentative de la graisse sous-cutanée.

Dans des modes de réalisation, sur chaque intervalle, la fonction d'approximation est une composition d'au moins une fonction polynomiale d'ordre deux.

Ces modes de réalisation permettent d'avoir une erreur très faible entre la fonction d'estimation et la courbe représentative tout en minimisant le temps de calcul pour déterminer la fonction d'approximation.

Dans des modes de réalisation, lors de l'étape de détermination, la courbe moyenne représentative de la graisse totale pour une coupe moyenne est déterminée, la coupe moyenne étant la coupe représentative de la moyenne de la graisse d'au moins deux coupes de l'image acquise.

Une telle moyenne permet de diminuer l'influence du bruit sur l'image acquise, pour une coupe.

Dans des modes de réalisation, chaque image acquise est dans une portion de la zone androïde du corps du corps vivant.

Ces modes de réalisation permettent de minimiser l'influence des os sur l'image acquise. De plus, la graisse viscérale est principalement située dans l'abdomen d'un corps vivant.

Dans des modes de réalisation, le procédé objet de l'invention comporte, de plus, une étape de conversion de la quantité de graisse viscérale calculée en masse de graisse viscérale et/ou en volume de graisse viscérale et/ou en aire moyenne de graisse viscérale.

Ces modes de réalisation permettent de modifier la forme de la donnée de quantité de graisse viscérale pour la comparer à d'autres méthodes d'estimation de la graisse viscérale.

Dans des modes de réalisation, le procédé objet de l'invention comporte, de plus, une étape de comparaison de la quantité de graisse convertie avec au moins une valeur limite prédéterminée.

Ces modes de réalisation permettent d'évaluer un risque de maladie cardiovasculaire du corps vivant.

Dans des modes de réalisation, au moins deux intervalles définis pour une coupe sont non vides.

Dans des modes de réalisation, l'image est acquise par absorptiométrie bi-photonique.

Ces modes de réalisation permettent de limiter le taux de radiation auquel est exposé le corps vivant. Une quantité de graisse viscérale peut être précisément estimée à partir d'imagerie par absorptiométrie bi-photonique. L'absorptiométrie bi-photonique (« dual-energy X-ray absorptiometry » d'acronyme « DXA » en anglais) est une technique d'imagerie capable d'estimer simultanément la quantité de tissus gras et de tissus maigres tout en les dissociant. Grâce à son faible taux de radiation, l'absorptiométrie bi-photonique est une très bonne alternative à l'imagerie par résonance magnétique et à la tomodensitométrie pour le suivi d'un corps vivant.

Selon un deuxième aspect, la présente invention vise un dispositif d'estimation de graisse viscérale d'un corps vivant, qui comporte :

- un moyen d'acquisition d'au moins une image planaire représentative d'une composition corporelle comportant l'abdomen du corps vivant,
- un moyen de détermination d'au moins une courbe représentative de la projection de la graisse totale dans l'image acquise suivant une coupe de l'image acquise,

pour chaque coupe :

- un moyen d'identification d'au moins quatre points d'intérêts définis tels que :

  - le premier point d'intérêt et le quatrième point d'intérêt correspondent au passage d'une valeur de graisse totale nulle à une valeur non nulle,
  - le deuxième point d'intérêt et le troisième point d'intérêt représentent un maximum local et
  - les courbes entre le premier point d'intérêt et le deuxième, et entre le troisième point d'intérêt et le quatrième, sont sensiblement des parties de projections d'une ellipse,

- un moyen de définition d'au moins deux intervalles entre le deuxième et le troisième point d'intérêt de la courbe,
- un moyen d'estimation, pour chaque intervalle, d'une fonction d'approximation de la graisse sous cutanée en fonction des variations attendues de la courbe représentative de la projection de la quantité de graisse totale et
- un moyen de calcul de la quantité de graisse viscérale comportant la soustraction de la fonction d'approximation de la graisse sous-cutanée à la courbe représentative de la projection de la graisse totale.

Les buts, avantages et caractéristiques particulières du dispositif objet de la présente invention étant similaires à ceux du procédé objet de la présente invention, ils ne sont pas rappelés ici.

BREVE DESCRIPTION DES FIGURES

D'autres avantages, buts et caractéristiques particulières de l'invention ressortiront de la description non limitative qui suit d'au moins un mode de réalisation particulier du dispositif et du procédé objets de la présente invention, en regard des dessins annexés, dans lesquels :

- la figure 1 représente, schématiquement et sous forme de logigramme, une succession d'étapes particulières du procédé objet de la présente invention,
- la figure 2 représente, schématiquement, une image de composition corporelle obtenue par absorptiométrie bi-photonique d'un corps d'un corps vivant ainsi que le positionnement des régions d'intérêt,
- la figure 3 représente, schématiquement, un élargissement de la zone androïde de l'image de composition corporelle obtenue par absorptiométrie bi-photonique d'un corps d'un corps vivant,
- la figure 4 représente, schématiquement, une courbe représentative de la projection de la graisse totale pour une coupe de l'image de composition corporelle, les points d'intérêt de cette courbe et une fonction d'approximation de la graisse sous-cutanée et
- la figure 5 représente, schématiquement, un dispositif objet de la présente invention.

DESCRIPTION D'EXEMPLES DE REALISATION DE L'INVENTION

La présente description est donnée à titre non limitatif, chaque caractéristique d'un mode de réalisation pouvant être combinée à toute autre caractéristique de tout autre mode de réalisation de manière avantageuse. Par ailleurs, chaque paramètre d'un exemple de réalisation peut être mis en oeuvre indépendamment d'autres paramètres dudit exemple de réalisation.

On note dès à présent que les figures ne sont pas à l'échelle.

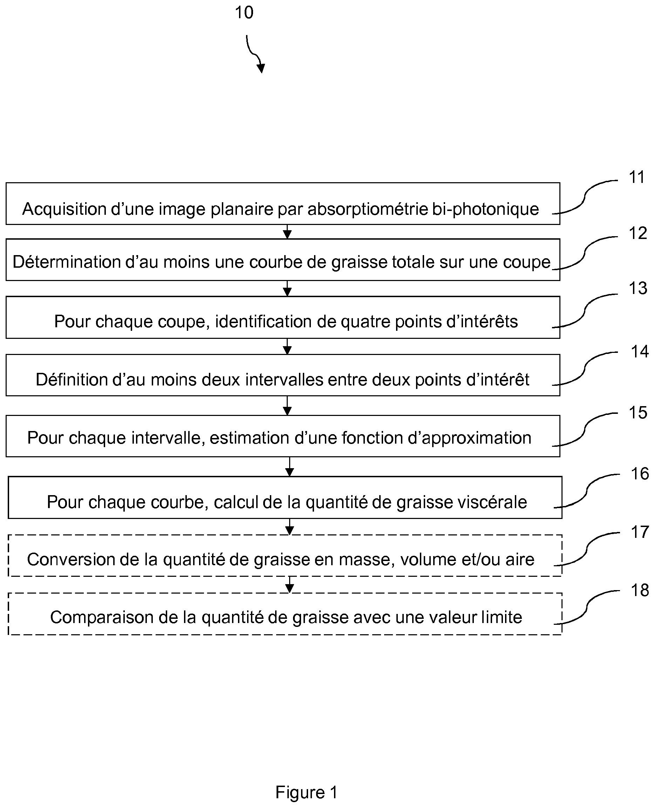

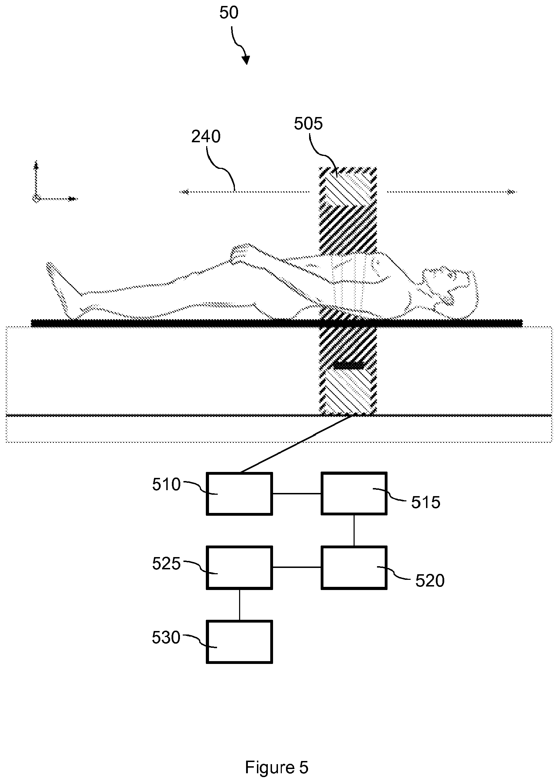

On observe, sur la figure 1, une vue schématique d'une succession d'étapes d'un mode de réalisation du procédé 10 objet de la présente invention. La description qui suit de la figure 1 est mise en oeuvre par un dispositif 50 représenté sur la figure 5.

Le procédé 10 d'estimation de graisse viscérale d'un corps vivant comporte une étape d'acquisition 11 d'au moins une image planaire représentative d'une composition corporelle comportant l'abdomen 30 du corps vivant. Le procédé 10 objet de la présente invention peut être mis en oeuvre à partir de toute image de composition corporelle d'un corps vivant. Préférentiellement, l'étape d'acquisition 11 est réalisée au moyen d'un absorptiomètre bi-photonique. Le moyen d'acquisition 505 du dispositif 50 est tout moyen d'acquisition d'une composition corporelle. Préférentiellement, le moyen d'acquisition 505 est un absorptiomètre bi-photonique.

Un absorptiomètre bi-photonique comprend une table sensiblement horizontale produisant une surface plane sur laquelle est allongé un corps vivant. Un absorptiomètre bi-photonique est également dénommé ostéodensitomètre. Sur la figure 5, un corps humain est représenté. L'acquisition d'une image 20 par absorptiométrie bi-photonique peut également être réalisée sur un objet ou un animal. L'absorptiomètre bi-photonique comporte un bras qui supporte une source de rayons X émettant un faisceau X. La source est positionnée sous la table de façon à être au plus près du corps vivant. Le bras est en forme de « C » et entoure la table. Les rayons X sont collimatés en un fin éventail ou en un pinceau à l'aide d'une pièce de plomb placée directement sur la source de rayons X. Afin de générer un faisceau X biénergie, un filtre de terre rare est placé entre le collimateur et le tube de rayons X (non représenté). Le filtre de terre rare sépare le spectre de rayons X en deux énergies différentes et différentiables, dépendantes de la terre rare utilisée.

Dans des modes de réalisation, la double énergie est réalisée en utilisant une alternation du niveau d'énergie de la source. La source de rayons X est dans des conditions de mesure où on assimile les rayons X à un faisceau perpendiculaire à la table. Au-dessus de la table, à l'extrémité supérieure du bras, est positionné un détecteur qui reçoit directement le rayonnement X produit par la source placée sous la table. Le détecteur est une succession d'éléments de Cadmium-Telluride (d'acronyme « CdT ») configurée pour discriminer les deux énergies.

La source et le détecteur sont alignés et permettent un mouvement synchronisé et simultané, parfaitement aligné, et dans toutes les directions de ces deux éléments. La source et le détecteur se déplacent linéairement, et solidairement l'un de l'autre, dans un plan parallèle à celui de la table.

Chaque ligne de scan correspond à un ensemble de pixels contenant l'information de la détection d'un rayon X, considérée comme linéaire entre la source et le détecteur. Chacune de ces lignes est associée à une coordonnée représentant la position du détecteur et de la source. Pour chaque pixel, le signal mesuré représente l'atténuation subie par le rayon X, sur le trajet source-détecteur, proportionnelle au matériau placé entre la source et le détecteur. L'atténuation dépend de la longueur et de la densité du matériau, par exemple.

La mesure est réalisée pour deux énergies distinctes, une haute énergie (d'acronyme « HE ») et une basse énergie (d'acronyme « BE »). Ainsi, pour chaque pixel du détecteur, le signal mesuré est une combinaison d'une atténuation haute énergie et d'une atténuation basse énergie. La discrimination des deux énergies peut être réalisée à l'aide d'un disque en rotation composé de deux atténuations venant couper le faisceau ou alors en utilisant un détecteur qui peut directement discriminer les deux énergies. Les mesures haute énergie et basse énergie sont post-traitées à l'aide de l'équation de Beer-Lambert afin d'obtenir les résultats de densité osseuse ou de composition corporelle. Les résultats étant composés d'une image de tissus gras, d'une image de tissus maigres et d'une image d'os.

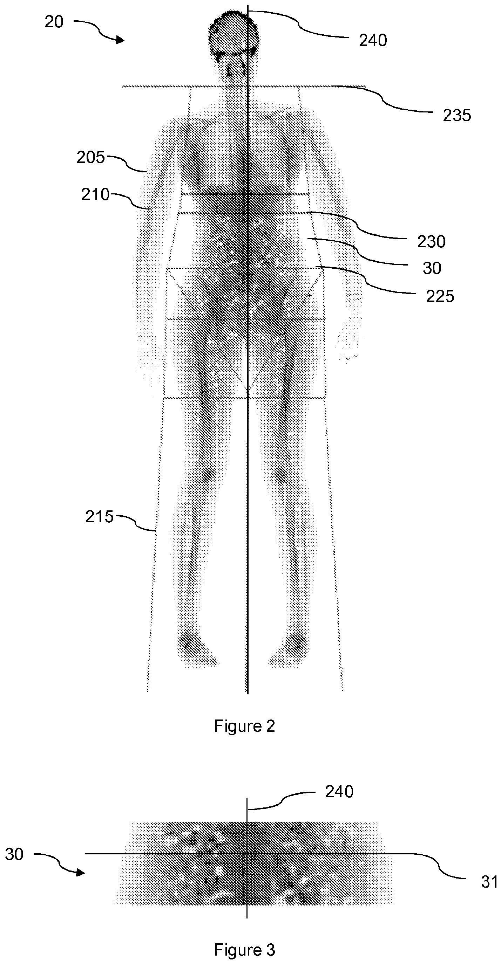

La figure 2 montre une image 20 par absorptiométrie bi-photonique d'un examen corps-entier d'un corps humain en position antéro-postérieure. Cette image est une projection en deux dimensions du corps vivant dont la composition corporelle est calculée et affichée suivant la méthode décrite ci-avant. L'image de composition corporelle est une combinaison d'une image osseuse 210, d'une image des tissus maigres et une image des tissus gras 205. Les informations sur les données osseuses ou sur les tissus peuvent être calculées séparément et un procédé mis en oeuvre par informatique peut être alors appliqué séparément sur chaque jeu de données, sur une région du corps vivant ou sur l'intégralité du corps vivant.

Des régions d'intérêt standards 215 sont superposées à l'examen du corps entier 20. Ces régions d'intérêt permettent au logiciel d'exploitation des données d'absorptiométrie bi-photonique de calculer, par exemple, la densité minérale osseuse (« Bone Minerai Density » en anglais d'acronyme « BMD »), le contenu minéral osseux (« Bone Minerai Content » en anglais d'acronyme « BMC ») et la composition tissulaire, dans des sous-sections du corps vivant.

Chacune des régions d'intérêt 215 est placée sur la représentation du corps du corps vivant à l'aide de marqueurs qui peuvent être, soit générés automatiquement, soit positionnés manuellement. Chaque marqueur et chaque région d'intérêt sont appliqués simultanément à l'image d'os et aux images des tissus. L'utilisateur peut ajuster manuellement la position des régions d'intérêt 215 pour que les régions d'intérêt 215 soient sensiblement alignées avec la morphologie du corps humain. Ce qui permet une meilleure estimation des points d'intérêts de la projection et, par suite, une meilleure estimation de la graisse viscérale.

La figure 3 montre un agrandissement d'une image de composition corporelle du corps vivant restreinte à une sous-région d'intérêt 30 définie de sorte à ce qu'elle soit incluse dans la région androïde. La région androïde est définie comme une portion de vingt pour cent de la distance de la crête iliaque 225 au menton 235. La région androïde est positionnée sur la ligne de la crête iliaque 225. La crête iliaque 225 est le bord supérieur de l'ilium, l'os le plus large présent dans la région pelvienne du corps humain. La sous-région d'intérêt 30 est placée de sorte qu'elle soit comprise dans la région androïde, au-dessus de la crête iliaque 225 et en dessous de la cage thoracique 230 de sorte qu'aucune côte ne soit présente dans la région d'intérêt 30. Il est alors possible de considérer que cette région d'intérêt est composée uniquement de tissus mous, une fois la colonne vertébrale exclue de l'image.

L'image acquise au cours de l'étape d'acquisition 11 est l'image d'au moins une portion de la région d'intérêt 30 contenue dans la région androïde du corps vivant. L'estimation de la graisse viscérale est effectuée, préférentiellement, dans une sous-région de la région d'intérêt 30 du corps vivant.

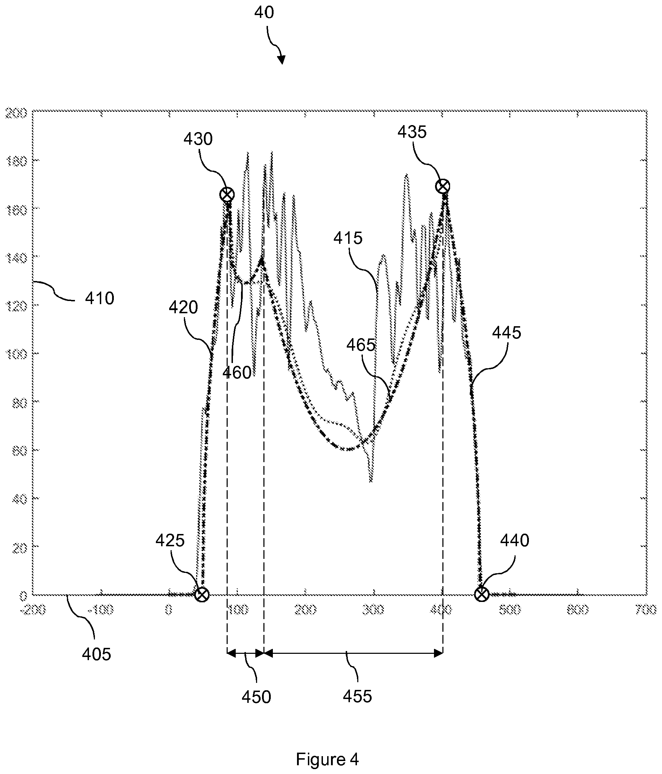

Le procédé 10 comporte une étape de détermination 12 d'au moins une courbe représentative 415 de la projection de la graisse totale dans l'image acquise 30 suivant une coupe 31 sur l'image acquise 30. La coupe 31 est une coupe perpendiculaire au plan de la table de l'absorptiomètre bi-photonique. On appelle coupe 31, une droite, sur l'image d'absorptiométrie bi-photonique représentative d'une coupe transverse du corps vivant. La projection de la graisse totale la coupe représentée par la droite 31 de la figure 3 est représenté par la courbe 415, en figure 4.

L'étape de détermination 12 est mise en oeuvre par un moyen de détermination 510 d'au moins une courbe représentative 415 de la projection de la graisse totale dans l'image acquise 20 suivant une coupe 31 sur l'image acquise 20.

La courbe 415 représente, la projection de la quantité de tissu gras, pour une coupe donnée 31, du corps vivant. La courbe 415 est représentée dans un repère orthonormé dont l'abscisse 405 représente un numéro de pixel sur la coupe 31 et l'ordonnée 410 représente la densité surfacique, en grammes par centimètres carrés, (g/cm$^2$), de tissus gras compris dans la coupe 31.

Ainsi, lorsque la coupe 31 n'est pas positionnée dans l'image 20, la courbe 415 est nulle. La courbe 415 présente une partie 420 sensiblement croissante.

Cette partie 420 représente la projection de graisse sous-cutanée au niveau du bord extérieur droit de l'abdomen du corps vivant jusqu'à la cavité abdominale du corps vivant. Dans le sens croissant de l'abscisse 405, la courbe 415 présente une partie 445 sensiblement décroissante jusqu'à une valeur nulle. Cette partie 445 représente la projection de graisse sous-cutanée entre la cavité abdominale et le bord extérieur gauche du corps vivant. Ces deux parties, 420 et 445, sont sensiblement symétriques par rapport à un axe parallèle à l'ordonnée. L'abdomen d'un corps vivant pouvant être associé à une ellipse, les deux parties, 420 et 445, sont assimilées des parties de la projection de l'ellipse représentative de l'abdomen du corps vivant sur la coupe 31.

Puis, le procédé 10 comporte, pour chaque coupe 31, une étape d'identification 13 d'au moins quatre points d'intérêts définis tels que :

- le premier point d'intérêt 425 et le quatrième point d'intérêt 440 correspondent au passage d'une valeur de graisse totale nulle à une valeur non nulle,
- le deuxième point d'intérêt 430 et le troisième point d'intérêt 435 représentent un maximum local et
- les courbes entre le premier point d'intérêt 425 et le deuxième 430, et entre le troisième point d'intérêt 435 et le quatrième 440, sont sensiblement des parties de projections d'une ellipse.

On rappelle ici que nulle, dans le cadre de projections de données réelles, définit une valeur très faible par rapport au reste des valeurs projetées. La valeur faible par rapport aux valeurs de graisse totale projetées peut représenter un bruit de mesure hors du corps vivant sur l'image acquise, par exemple. En d'autres termes, le bruit de mesure hors du corps vivant est négligé.

L'étape d'identification 13 positionne, sur la courbe 415, les points d'intérêts représentatifs de la projection de graisse sous-cutanée du corps vivant entre le bord extérieur du corps vivant et la cavité abdominale sur la coupe 31. Les points d'intérêt, 425, 430, représentent le début et la fin de la partie 420 de la courbe 415. Les points d'intérêt, 435 et 440, représentent le début et la fin de la partie 445 de la courbe 415.

Le point 425 est obtenu par recherche de gauche à droite du premier point supérieur à une première valeur limite prédéterminée dépendante du corps vivant, la valeur limite prédéterminée correspondant au bruit de mesure hors du corps vivant, et dont les points suivants sont de valeur non nulle supérieure à la première valeur limite prédéterminée.

Le point 440 est obtenu par recherche de droite à gauche du dernier point supérieur à la première valeur limite prédéterminée et dont les points précédents sont de valeur non nulle supérieure à la première valeur limite prédéterminée.

Le point 430 est le premier maximum local positionné après le point 425 dont l'amplitude est supérieure à une deuxième valeur limite prédéterminée dépendante du corps vivant définie en fonction de la déviation standard des valeurs de projection de graisse comprises entre les points 425 et 440.

Le point 435 est le dernier maximum local positionné avant le point 440 dont l'amplitude est supérieure à la deuxième valeur limite prédétermine définie en fonction de la déviation standard des valeurs de projection de graisse comprises entre les points 425 et 440.

Les maximums locaux peuvent être représentés par une variation significative d'intensité du niveau de graisse sur l'image acquise visible sur au moins une coupe 31. La variation significative du niveau de graisse peut être représentée, sur l'image 30, par une variation du niveau de gris représentatif de la graisse et visible par un opérateur du dispositif 50.

Dans des modes de réalisation, l'identification des points d'intérêts, 425, 430, 435 et 440, est effectué par identification d'un maximum local sur la courbe 415 correspondant à une démarcation visible sur l'image acquise 30. Le maximum local est alors pris comme point d'intérêt.

L'identification des points d'intérêt, 425, 430, 435 et 440, permet, entre le deuxième point d'intérêt 430 et le troisième point d'intérêt 435, d'estimer la partie de l'abdomen du corps vivant dans laquelle se situe la graisse viscérale, c'est-à-dire à l'intérieur de la cavité abdominale.

L'étape d'identification 13 est mise en oeuvre par un moyen d'identification 515 d'au moins quatre points d'intérêts, 425, 430, 435 et 440.

Une fois les points d'intérêt identifiés, le procédé 10 comporte une étape de définition 14 d'au moins deux intervalles, 450 et 455, entre le deuxième 430 et le troisième point d'intérêt 435 de la courbe 415.

Le procédé 10, une fois les points 430 et 435 identifiés, lisse la courbe 415 représentative de la projection de graisse entre ces deux points. Le procédé de lissage est accentué afin d'obtenir une tendance globale de la projection de graisse. Puis, le procédé 10 recherche les points de variations de cette fonction lissée. L'ensemble de ces points défini le nombre d'intervalles de la projection. Si aucun point n'est trouvé alors il est considéré que l'un des intervalles est vide.

Les paramètres physiologiques du corps vivant influencent la position des intervalles, 450 et 455, mais aussi l'amplitude de graisse sous-cutanée attendue sur ces intervalles.

Dans des modes de réalisation, au moins deux intervalles, 450 et 455, définis pour une coupe 31 sont non vides.

L'étape de définition 14 peut être mise en oeuvre par un moyen de définition 520 d'au moins deux intervalles, 450 et 455, entre le deuxième 430 et le troisième 435 point d'intérêt de la courbe 415.

Pour chaque intervalle, 450 et 455, défini pour une coupe 31, le procédé 10 comporte une étape d'estimation 15 d'une fonction d'approximation, 460, 465, de la graisse sous-cutanée en fonction des variations attendues de la courbe 415 représentative de la quantité de graisse totale.

Préférentiellement, chaque intervalle, 450 et 455, est compris entre les abscisses des deuxième 430 et troisième 435 points d'intérêts.

En fonction de la morphologie du corps vivant, les maximums locaux 430 et 435 peuvent former une partie sensiblement constante de la courbe 415. Dans ce cas, la position de cette partie sensiblement constante est utilisée pour définir les maximums locaux 430 et 435.

Afin d'approximer au mieux la courbe représentative de la projection de graisse sous-cutanée, les éléments suivants sont repérés : le nombre d'intervalles, la position de chaque point d'intérêt définissant les intervalles sur l'axe des abscisses, l'ordonnée de chacun de ces points, ainsi que les variations attendues de la projection dans ces intervalles, 450 et 455. En fonction des éléments repérés, le jeu de paramètres d'approximation des fonctions, sur chaque intervalle, 450 et 455, représentant la projection de graisse sous-cutanée est estimé. La définition des paramètres d'approximation est fondée sur une analyse statistique faite sur la segmentation de graisse sous-cutanée et totale sur une centaine de corps humains.

Préférentiellement, sur chaque intervalle, 450 et 455, la fonction d'approximation est une composition d'au moins une fonction polynomiale. Dans des modes de réalisation, le degré du polynôme peut être limité et, en fonction du degré du polynôme, le nombre d'intervalles peut être prédéterminé.

Préférentiellement, sur chaque intervalle, 450 et 455, la fonction d'approximation est une fonction polynomiale d'ordre deux.

Préférentiellement, l'étape d'estimation 15 est mise en oeuvre par un moyen d'estimation 525 d'une fonction d'approximation, 460 et 465, des variations attendues de la courbe représentative 415 de la projection de la quantité de graisse totale, sur chaque intervalle, 450 et 455.

Le procédé 10 comporte une étape de calcul 16 de la quantité de graisse viscérale comportant la soustraction de la fonction d'approximation, 420, 445, 460 et 465, à la courbe représentative 415 de la projection de graisse totale.

Le calcul de la quantité de la graisse viscérale est l'intégrale de la soustraction de la fonction d'approximation de la graisse sous-cutanée 420, 445 460 et 465 à la courbe 415 représentative de la projection de la graisse totale. L'unité de la quantité de graisse viscérale calculée est alors la densité linéique (g/cm).

Dans des modes de réalisation, lors de l'étape de détermination 12, la courbe moyenne représentative 415 de la graisse totale pour une coupe moyenne est déterminée, la coupe moyenne étant la coupe représentative de la graisse moyenne d'au moins deux coupes 31 de l'image acquise 30.

Lorsque le corps vivant est pas ou peu incliné, toutes courbes représentatives des projections selon les coupes perpendiculaires à l'axe 240 sont ajoutées les unes aux autres, la projection totale est ensuite divisée par le nombre de coupes. La coupe moyenne est ainsi obtenue. La région d'intérêt étant petite, l'inclinaison du corps vivant est négligeable. Lorsque le corps vivant est incliné, toutes les courbes représentatives des projections selon les coupes perpendiculaires à l'axe 240 sont ajoutées les unes aux autres en prenant en compte l'inclinaison du corps vivant.

Dans ces deux cas, les points d'intérêts sont calculés sur la coupe moyenne.

Ces modes de réalisation permettent de limiter le risque d'erreur dû au bruit lors de l'acquisition de l'image 30.

Ensuite, par hypothèse, l'étape de calcul 16 comporte une étape d'imposition de la valeur de cette soustraction comme étant nulle entre les points 425 et 430, et entre 435 et 440. La courbe ainsi obtenue est la projection de graisse viscérale (en g/cm$^2$). L'étape de calcul 16 comporte une étape d'addition de l'ensemble des points de la courbe obtenue et une étape de multiplication de la somme obtenue par la taille d'un pixel suivant l'axe 405. L'étape de calcul 16 résulte en l'obtention d'une densité linéique de graisse viscérale (g/cm) pour une coupe.

Lorsque l'étape de calcul 16 est effectuée coupe par coupe, la densité linéique obtenue est additionnée pour l'ensemble des coupes et multipliée par la taille verticale d'un pixel suivant l'axe 240 de l'image 20 pour obtenir une masse.

Lorsqu'une coupe moyenne est déterminée au préalable, la densité linéique est multipliée par le nombre de coupes et par la taille verticale d'un pixel suivant l'axe 240, pour obtenir une masse.

Dans les modes de réalisation dans lesquels l'étape de calcul 16 est réalisée sur une courbe représentative de la projection de graisse totale inclue dans la région d'intérêt 30 ou sur une courbe représentative de la projection moyenne de graisse totale inclue dans la région d'intérêt 30.

Pour une coupe moyenne composée d'un ensemble de N coupes, la masse de graisse viscérale est obtenue en utilisant la formule suivante :

$$M_{VAT} = T_{pix,y}[\langle F_{tot}\rangle - \langle F_{sat}\rangle] * N \qquad \text{A1}$$

Dans le cas du calcul de la masse de graisse viscérale sur N coupes indépendantes dans un volume l'équation est :

$$M_{VAT} = T_{pix,y}\left[\sum_{i=1}^{N}[F_{tot,i} - F_{sat,i}]\right] \qquad \text{A2}$$

Avec $M_{VAT}$ la masse de graisse viscérale, $T_{pix,y}$ la taille d'un pixel suivant l'axe 240 (en cm), $F_{TOT}$ la densité linéique de graisse totale présente dans la coupe (en g/cm), <Ftot> la densité linéique de graisse totale présente dans la coupe moyenne (en g/cm), $F_{SAT}$ la densité linéique de graisse sous-cutanée estimée (en g/cm) sur la dite coupe et <Fsat> la densité linéique de graisse sous-cutanée estimée (en g/cm) sur la dite coupe.

Et, de façon similaire, dans les modes de réalisation dans lesquels l'étape de calcul 16 est réalisée sur une courbe représentative de la projection de graisse totale inclue dans la région d'intérêt 30 ou sur une courbe représentative de la projection moyenne de graisse totale inclue dans la région d'intérêt 30.

Pour une coupe moyenne composée d'un ensemble de N coupes, le volume ($V_{VAT}$) de graisse viscérale est obtenu en utilisant la formule B1 ou B2 suivante :

$$V_{VAT} = T_{pix,y}[\langle F_{tot}\rangle - \langle F_{sat}\rangle] * N/D_g \qquad \text{B1}$$

Dans le cas du calcul du volume de graisse viscérale sur N coupes indépendantes dans un volume l'équation est :

$$V_{VAT} = T_{pix,y}\left[\sum_{i=1}^{N}[F_{tot,i} - F_{sat,i}]\right]/D_g \qquad \text{B2}$$

Avec $D_g$ la densité volumique de graisse (en g/cm$^3$).

Finalement, pour une coupe moyenne composée d'un ensemble de N coupes, l'aire moyenne ($A_{VAT}$) de graisse viscérale est obtenue en utilisant la formule C1 ou C2 suivante :

$$A_{VAT} = [(F_{tot}) - (F_{sat})]/D_g$$

<div align="right">C1</div>

Dans le cas du calcul de l'aire de graisse viscérale sur N coupes indépendantes dans un volume l'équation est :

$$A_{VAT} = \frac{1}{ND_g}\left[\sum_{i=1}^{N}\left[F_{tot,i} - F_{sat,i}\right]\right]$$

<div align="right">C2</div>

Dans des modes de réalisation, le procédé 10 comporte, de plus, une étape de conversion 17 de la quantité de graisse viscérale calculée en masse de graisse viscérale et/ou en volume de graisse viscérale et/ou en aire de graisse viscérale. L'étape de conversion 17 est réalisée au moyen de l'une des formules A1, A2, B1, B2, C1 ou C2.

Dans des modes de réalisation, le procédé 10 comporte une étape de comparaison 18 de la quantité de graisse convertie avec au moins une valeur limite prédéterminée. La valeur limite prédéterminée peut être :

- représentative d'une mesure effectuée au moyen d'un autre dispositif d'évaluation de la graisse viscérale,
- représentative d'une valeur limite telle que, lorsque la quantité de graisse viscérale est supérieure à la valeur limite prédéterminée, la personne présente un risque dû à cette quantité de graisse viscérale, tel que, par exemple, un risque accru de maladie cardiovasculaire.

L'ensemble de ces paramètres permet d'estimer complètement la graisse viscérale, et, en utilisant le même jeu d'équations, il est possible d'estimer des paramètres correspondants à la graisse sous-cutanée. L'ensemble de ces paramètres peuvent être affichés séparément ou suivant différentes combinaisons. La quantité de graisse viscérale estimée peut être utilisée lors d'un suivi afin d'évaluer la progression de la proportion de graisse viscérale et de graisse sous-cutanée lors d'une période de régime, de traitement ou d'activité physique.

La quantité de graisse sous-cutanée estimée ainsi que la quantité de graisse viscérale estimée sont aussi incluses dans des rapports de composition corporelle au même titre que la densité osseuse. La présentation simultanée de la graisse viscérale et de la graisse sous-cutanée permet à l'utilisateur de suivre le pourcentage de graisse viscérale et ainsi de suivre le risque de maladie cardiovasculaire, par exemple.

On observe sur la figure 5, un dispositif 50 d'estimation de graisse viscérale d'un corps vivant qui comporte :

- un moyen d'acquisition 505 d'au moins une image planaire 30 représentative d'une composition corporelle comportant l'abdomen du corps vivant,
- un moyen de détermination 510 d'au moins une courbe représentative 415 de la projection de graisse totale dans l'image acquise suivant une coupe sur l'image acquise, pour chaque coupe :
- un moyen d'identification 515 d'au moins quatre points d'intérêts, 425, 430, 435, 440, définis tels que :

  - le premier point d'intérêt 425 et le quatrième 440 point d'intérêt correspondent au passage d'une valeur de graisse totale nulle à une valeur non nulle,
  - le deuxième point d'intérêt 430 et le troisième point 435 d'intérêt représentent un maximum local et
  - les courbes entre le premier point d'intérêt 425 et le deuxième 430, et entre le troisième point 435 d'intérêt et le quatrième 440, sont sensiblement des projections de parties d'une ellipse,

- un moyen de définition 520 d'au moins deux intervalle, 450 et 455, entre le deuxième 430 et le troisième 435 point d'intérêt de la courbe,
- pour chaque intervalle, 450 et 455, un moyen d'estimation 525 d'une fonction d'approximation, 460 et 465, de la graisse sous cutanée en fonction de variations attendues de la courbe représentative de la projection de la quantité de graisse totale et
- un moyen de calcul 530 de la quantité de graisse viscérale comportant la soustraction de la fonction d'approximation de la graisse sous-cutanée à la courbe représentative de la projection de la graisse totale.

Les moyens de détermination 510, d'identification 515, de définition 520, d'estimation 525, et de calcul 530 sont préférentiellement un microprocesseur mettant en oeuvre un programme informatique stocké sur une mémoire vive.

Pour résumer, le procédé 10 décrit ci-dessus comprend la réalisation d'un examen d'un corps vivant qui fournit des données de composition corporelle. L'image du corps entier contient différentes régions d'intérêt utilisées lors du calcul de la graisse viscérale et de la graisse sous-cutanée ainsi que d'autres paramètres spécifiques au corps entier. L'algorithme utilise ensuite ces régions d'intérêt afin de séparer la région androïde en plusieurs sous parties et en sélectionne spécifiquement une excluant la crête iliaque et les côtes. Cette région d'intérêt est composée d'une image os, d'une image graisse et d'une image tissus maigres. Sur ces images, la méthode décrite sélectionne un jeu de paramètres permettant l'estimation, aussi précise que possible, de la répartition de graisse sous-cutanée. Finalement, la graisse sous-cutanée est soustraite à la graisse totale permettant l'estimation de la graisse viscérale. Les résultats de graisse viscérale et de graisse sous-cutanée peuvent alors être affichés en termes de masse, d'aire et de volume. Ces résultats peuvent être inclus dans des rapports concernant le corps entier du corps vivant.

**Revendications**

1. Procédé (10) d'estimation de graisse viscérale d'un corps vivant, qui comporte les étapes suivantes :

   - acquisition (11) d'au moins une image planaire (30) représentative d'une composition corporelle comportant l'abdomen du corps vivant,
   - détermination (12) d'au moins une courbe représentative (415) de la projection de de la graisse totale dans l'image acquise suivant une coupe (31) sur l'image acquise,

   pour chaque coupe :

   - identification (13) d'au moins quatre points d'intérêts (425, 430, 435, 440) définis tels que :

      - le premier point d'intérêt (425) et le quatrième point d'intérêt (440) correspondent au passage d'une valeur de graisse totale nulle à une valeur non nulle,
      - le deuxième point d'intérêt (430) et le troisième point d'intérêt (435) représentent un maximum local et
      - les courbes entre le premier point d'intérêt et le deuxième, et entre le troisième point d'intérêt et le quatrième, sont sensiblement des parties de projections d'une ellipse,

   - définition (14) d'au moins deux intervalles (450, 455) entre le deuxième et le troisième point d'intérêt de la courbe,
   - pour chaque intervalle, estimation (15) d'une fonction d'approximation (460, 465) de la graisse sous cutanée en fonction des variations attendues de la courbe représentative de la projection de la quantité de graisse totale,
   - calcul (16) de la quantité de graisse viscérale comportant la soustraction de la fonction d'approximation de la graisse sous-cutanée à la courbe représentative de la projection de la graisse totale.

2. Procédé (10) selon la revendication 1, dans lequel, sur chaque intervalle (450, 455), la fonction d'approximation (460, 465) est une composition d'au moins une fonction polynomiale.

3. Procédé (10) selon la revendication 2, dans lequel, sur chaque intervalle (450, 455), la fonction d'approximation (460, 465) est une composition d'au moins une fonction polynomiale d'ordre deux.

4. Procédé (10) selon l'une des revendications 1 à 3, dans lequel lors de l'étape de détermination (12), la courbe moyenne représentative (415) de la graisse totale pour une coupe moyenne est déterminée, la coupe moyenne étant la coupe représentative de la moyenne de la graisse d'au moins deux coupes (31) de l'image acquise (30).

5. Procédé (10) selon l'une des revendications 1 à 4, dans lequel chaque image acquise (30) est dans une portion de la zone androïde du corps du corps vivant.

6. Procédé (10) selon l'une des revendications 1 à 5, qui comporte, de plus, une étape de conversion (17) de la quantité de graisse viscérale calculée en masse de graisse viscérale et/ou en volume de graisse viscérale et/ou en aire moyenne de graisse viscérale.

7. Procédé (10) selon la revendication 6, qui comporte, de plus, une étape de comparaison (18) de la quantité de graisse convertie avec au moins une valeur limite prédéterminée.

8.  Procédé (10) selon l'une des revendications 1 à 7, dans lequel, au moins deux intervalles (450, 455) définis pour une coupe (31) sont non vides.

9.  Procédé (10) selon l'une des revendications 1 à 8, dans lequel, l'image est acquise par absorptiométrie bi-photonique.

10. Dispositif (50) d'estimation de graisse viscérale d'un corps vivant, qui comporte:

    - un moyen d'acquisition (505) d'au moins une image planaire (30) représentative d'une composition corporelle comportant l'abdomen du corps vivant,
    - un moyen de détermination (510) d'au moins une courbe représentative (415) de la projection de la graisse totale dans l'image acquise suivant une coupe (31) sur l'image acquise,

    pour chaque coupe :

    - un moyen d'identification (515) d'au moins quatre points d'intérêts (425, 430, 435, 440) définis tels que :

        - le premier point d'intérêt (425) et le quatrième point d'intérêt (440) correspondent au passage d'une valeur de graisse totale nulle à une valeur non nulle,
        - le deuxième point d'intérêt (430) et le troisième point d'intérêt (435) représentent un maximum local et
        - les courbes entre le premier point d'intérêt et le deuxième, et entre le troisième point d'intérêt et le quatrième, sont sensiblement des parties de projections d'une ellipse,

    - un moyen de définition (520) d'au moins deux intervalles (450, 455) entre le deuxième et le troisième point d'intérêt de la courbe,
    - un moyen d'estimation (525), pour chaque intervalle, d'une fonction d'approximation (460, 465) de la graisse sous cutanée en fonction des variations attendues de la courbe représentative de la projection de la quantité de graisse totale,
    - un moyen de calcul (530) de la quantité de graisse viscérale comportant la soustraction de la fonction d'approximation de la graisse sous-cutanée à la courbe représentative de la projection de la graisse totale.

**Patentansprüche**

1.  Verfahren (10) zum Einschätzen des viszeralen Fetts eines lebenden Körpers, das die folgenden Schritte umfasst:

    - Aufnehmen (11) wenigstens eines planaren Bildes (30), das eine Körperzusammensetzung darstellt, die den Unterleib des lebenden Körpers umfasst,
    - Bestimmung (12) wenigstens einer Kurve (415), die die Projektion des gesamten Fettes in dem in dem aufgenommenen Bild gemäß einem Schnitt (31) auf dem aufgenommenen Bild darstellt,

    für jeden Schnitt;

    - Identifizierung (13) von wenigstens vier definierten wichtigen Punkten (425, 430, 435, 440), wie z. B.:

        - der erste wichtige Punkt (425) und der vierte wichtige Punkt (440) entsprechen dem Übergang eines Gesamtfett-Wertes von Null zu einem Wert von ungleich Null,
        - der zweite wichtige Punkt (430) und der dritte wichtige Punk (435) stellen ein örtliches Maximum dar und
        - die Kurven zwischen dem ersten und dem zweiten wichtigen Punkt und zwischen dem dritten und dem vierten wichtigen Punkt sind deutlich Teile von Projektionen einer Ellipse,

    - Definition (14) von wenigstens zwei Intervallen (450, 455) zwischen dem zweiten und dem dritten wichtigen Punkt der Kurve,
    - für jedes Intervall Einschätzung (15) einer Annäherungsfunktion (460, 465) des subkutanen Fetts in Abhängigkeit von den erwarteten Schwankungen der Kurve, die die Projekte der Gesamtfett-Menge darstellt,
    - Berechnung (16) der viszeralen Fettmenge, umfassend die Subtraktion der Einschätzungsfunktion des subkutanen Fetts von der Kurve, die die Projektion des gesamten Fetts darstellt.

2.  Verfahren (10) gemäß Anspruch 1, bei dem die Einschätzungsfunktion (460, 465) bei jedem Intervall (450, 455) eine Zusammensetzung aus wenigstens einer polynomischen Funktion ist.

3. Verfahren (10) gemäß Anspruch 2, bei dem die Einschätzungsfunktion (460, 465) bei jedem Intervall (450, 455) eine Zusammensetzung aus wenigstens einer polynomischen Funktion der Ordnung zwei ist.

4. Verfahren (10) gemäß einem der Ansprüche 1 bis 3, bei dem bei dem Bestimmungsschritt (12) die durchschnittliche Kurve (415), die das gesamte Fett darstellt, für einen durchschnittlichen Schnitt bestimmt ist, wobei der durchschnittliche Schnitt die Kurve ist, die das Mittel des Fetts aus wenigstens zwei Schnitten (31) des aufgenommenen Bildes (30) ist, bestimmt wird.

5. Verfahren (10) gemäß einem der Ansprüche 1 bis 4, bei dem jedes Bild (30) in einem Abschnitt des androiden Bereichs des Körpers des lebenden Körpers aufgenommen wird.

6. Verfahren (10) gemäß einem der Ansprüche 1 bis 5, das darüber hinaus einen Umwandlungsschritt (17) der viszeralen Fettmenge umfasst, die in der viszeralen Fettmenge und / oder im viszeralen Fettvolumen und / oder im durchschnittlichen viszeralen Fettareal berechnet wird.

7. Verfahren (10) gemäß Anspruch 9, das darüber hinaus einen Vergleichsschritt (18) der umgewandelten Fettmenge mit wenigstens einem vorbestimmten Grenzwert umfasst.

8. Verfahren (10) gemäß einem der Ansprüche 1 bis 7, bei dem wenigstens zwei Intervalle (450, 455), die für einen Schnitt (31) definiert werden, nicht leer sind.

9. Verfahren (10) gemäß einem der Ansprüche 1 bis 8, bei dem das Bild per biphotonischer Absorptiometrie erworben wird.

10. Vorrichtung (50) zum Einschätzen des viszeralen Fetts eines lebenden Körpers, **dadurch gekennzeichnet, dass** sie umfasst:

- ein Mittel zum Aufnehmen (505) wenigstens eines planaren Bildes (30), das eine Körperzusammensetzung darstellt, die den Unterleib des lebenden Körpers umfasst,
- ein Mittel zur Bestimmung (510) wenigstens einer Kurve (415), die die Projektion des gesamten Fettes in dem in dem aufgenommenen Bild gemäß einem Schnitt (31) auf dem aufgenommenen Bild darstellt,

für jeden Schnitt;

- ein Mittel zur Identifizierung (515) von wenigstens vier definierten wichtigen Punkten (425, 430, 435, 440), wie z. B.:

- der erste wichtige Punkt (425) und der vierte wichtige Punkt (440) entsprechen dem Übergang eines Gesamtfett-Wertes von Null zu einem Wert von ungleich Null,
- der zweite wichtige Punkt (430) und der dritte wichtige Punk t(435) stellen ein örtliches Maximum dar und
- die Kurven zwischen dem ersten und dem zweiten wichtigen Punkt und zwischen dem dritten und dem vierten wichtigen Punkt sind deutlich Teile von Projektionen einer Ellipse,

- ein Mittel zur Definition (520) von wenigstens zwei Intervallen (450, 455) zwischen dem zweiten und dem dritten wichtigen Punkt der Kurve,
- für jedes Intervall ein Mittel zur Einschätzung (525) einer Annäherungsfunktion (460, 465) des subkutanen Fetts in Abhängigkeit von den erwarteten Schwankungen der Kurve, die die Projektion der Gesamtfett-Menge darstellt,
- ein Mittel zur Berechnung (530) der viszeralen Fettmenge, umfassend die Subtraktion der Einschätzungsfunktion des subkutanen Fetts von der Kurve, die die Projektion des gesamten Fetts darstellt.

## Claims

1. Method (10) of estimating the visceral fat of a living body, which comprises the following steps:

- acquiring (11) at least one planar image (30) representative of a body composition including the abdomen of the living body;
- determining (12) at least one curve (415) representative of the projection of the total fat in the acquired image along a cross-section (31) on the acquired image;

for each cross-section:

- identifying (13) at least four points of interest (425, 430, 435, 440) defined such that:

- the first point of interest (425) and the fourth point of interest (440) correspond to the transition from a zero value for total fat to a non-zero value;
- the second point of interest (430) and the third point of interest (435) represent a local maximum; and
- the curves between the first and second points of interest, and between the third and fourth points of interest, are substantially projection portions of an ellipse;

- defining (14) at least two intervals (450, 455) between the second and third points of interest of the curve;
- for each interval, estimating (15) a function (460, 465) for approximating the subcutaneous fat as a function of expected variations in the curve representative of the projection of the total amount of fat;
- calculating (16) the amount of visceral fat including removing the subcutaneous fat approximation function from the curve representative of the projection of the total fat.

2. Method (10) according to claim 1 wherein, over each interval (450, 455), the approximation function (460, 465) is a composition of at least one polynomial function.

3. Method (10) according to claim 2 wherein, over each interval (450, 455), the approximation function (460, 465) is a composition of at least one second-order polynomial function.

4. Method (10) according to one of claims 1 to 3 wherein, during the determination step (12), the average curve (415) representative of the total fat for an average cross-section is determined, the average cross-section being the cross-section representative of the average fat of at least two cross-sections (31) of the acquired image (30).

5. Method (10) according to one of claims 1 to 4, wherein each acquired image (30) is in a portion of the living body's android region of the body.

6. Method (10) according to one of claims 1 to 5, which also comprises a step (17) of converting the calculated amount of visceral fat into visceral fat mass and/or visceral fat volume and/or average visceral fat area.

7. Method (10) according to claim 6, which also comprises a step (18) of comparing the converted fat amount to at least one predefined limit value.

8. Method (10) according to one of claims 1 to 7, wherein at least two defined intervals (450, 455) for a cross-section (31) are not empty.

9. Method (10) according to one of claims 1 to 8, wherein the image is acquired by dual-photon absorptiometry.

10. Device (50) for estimating the visceral fat of a living body, **characterized in that** it comprises:

- a means (505) for acquiring at least one planar image (30) representative of a body composition including the abdomen of the living body;
- a means (510) for determining at least one curve (415) representative of the projection of the total fat in the acquired image along a cross-section (31) on the acquired image;

for each cross-section:

- a means (515) for identifying at least four points of interest (425, 430, 435, 440) defined such that:

- the first point of interest (425) and the fourth point of interest (440) correspond to the transition from a zero value for total fat to a non-zero value;

- the second point of interest (430) and the third point of interest (435) represent a local maximum; and
- the curves between the first and second points of interest, and between the third and fourth points of interest, are substantially projection portions of an ellipse;

- a means (520) for defining at least two intervals (450, 455) between the second and third points of interest of the curve;
- a means (525) for estimating, for each interval, a function (460, 465) for approximating the subcutaneous fat as a function of expected variations in the curve representative of the projection of the total amount of fat;
- a means (530) for calculating the amount of visceral fat including removing the subcutaneous fat approximation function from the curve representative of the projection of the total fat.

10

| Acquisition d'une image planaire par absorptiométrie bi-photonique | 11 |
| Détermination d'au moins une courbe de graisse totale sur une coupe | 12 |
| Pour chaque coupe, identification de quatre points d'intérêts | 13 |
| Définition d'au moins deux intervalles entre deux points d'intérêt | 14 |
| Pour chaque intervalle, estimation d'une fonction d'approximation | 15 |
| Pour chaque courbe, calcul de la quantité de graisse viscérale | 16 |
| Conversion de la quantité de graisse en masse, volume et/ou aire | 17 |
| Comparaison de la quantité de graisse avec une valeur limite | 18 |

Figure 1

Figure 2

Figure 3

Figure 4

Figure 5

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 9179873 B
- US 8483458 B
- US 8300911 B